# EUROPÄISCHE PATENTSCHRIFT

(11) **EP 3 771 907 B1**
(45) Veröffentlichungstag und Bekanntmachung des Hinweises auf die Patenterteilung: **28.06.2023**
(21) Anmeldenummer: 20188633.0
(22) Anmeldetag: 30.07.2020
(51) Int. Cl.: G01N 33/38, G01N 27/04

(54) **VERFAHREN ZUR BESTIMMUNG DES FEUCHTEGEHALTS VON BETON UND ESTRICH**
METHOD FOR DETERMINING THE MOISTURE CONTENT OF CONCRETE AND SCREED
PROCÉDÉ DE DÉTERMINATION DE LA TENEUR EN HUMIDITÉ DU BÉTON ET DE LA CHAPE

(30) Priorität: 31.07.2019 DE 102019211411
(43) Veröffentlichungstag der Anmeldung: 03.02.2021
(73) Patentinhaber: Fraunhofer-Gesellschaft zur Förderung der angewandten Forschung e.V., 80686 München (DE)
(72) Erfinder: Kaufmann, Andreas, 83626 Valley (DE); Zillig, Wolfgang, 83626 Valley (DE)
(74) Vertreter: Friese Goeden Patentanwälte PartGmbB

(56) Entgegenhaltungen:
- WO-A1-2020/208471
- CN-A- 109 752 416
- CN-U- 208 060 432
- DE-A1-102013 018 917

## Beschreibung

Die Erfindung betrifft ein Verfahren zur Bestimmung des Feuchtegehalts von Beton und Estrich sowie eine Vorrichtung, die dazu verwendet werden kann.

Als Estrich bezeichnet man den Aufbau des Fußbodens als Untergrund für Fußbodenbeläge. Er ist zumindest in westlichen Ländern ein Standardbauteil von Gebäuden und befindet sich in allen Räumen und Zimmern. Eine gebräuchliche Ausführung im Massivbau besteht aus einem hydraulisch härtendem Zementestrich.

Ein solcher Zementestrich benötigt Wasser zum Aushärten. Solche Estriche werden also immer feucht oder nass eingebaut. Da der Estrich als Untergrund für einen Fußbodenbelag dient, wird er mit einer Deckschicht versehen, nachdem er seine "Belegereife" erreicht hat. "Belegereife" bedeutet, dass der Estrich, je nach geplantem Belag ausreichend getrocknet sein muss. Die Werte für die zulässige Restfeuchte bis zur Belegereife sind abhängig von der Estrichart, von der unbeheizten oder beheizten Konstruktion und von der Art des späteren Belags.

Die Ermittlung der Estrich-Restfeuchte erfolgt bis heute nach der so genannten CM-Methode, die sehr aufwändig ist.

Dafür muss nach einer gewissen Zeitspanne, die der Bodenleger für ausreichend hält, ein Loch in den verlegten Estrich geschlagen werden. Das so gewonnene Estrich-Material wir dann in ein Calciumcarbid-Messgerät (CM-Gerät) gegeben und über ein chemisches Verfahren die enthaltene Rest-Feuchte ermittelt. Dieses Verfahren ist nicht nur sehr zeitaufwändig. Es ist zudem nicht zerstörungsfrei und liefert keine Informationen über den Trocknungszustand des Estrichs in Abhängigkeit der Tiefe.

Estrich trocknet in Abhängigkeit der herrschenden Lufttemperatur und relativen Feuchte unterschiedlich schnell. Trocknet er zu schnell, kann es sein, dass er seine Endfestigkeit noch nicht erreicht hat, trocknet er zu langsam, könnte er schon zu früh mit einem Belag versehen werden, was zu späteren Feuchteschäden führen kann (z.B. aufquellendes Parkett).

Da verlegter Estrich nur mit seiner Oberfläche an die Raumluft angrenzt, ist der hauptsächliche Austragspfad für die Feuchte seine raumseitige Oberfläche. Der Estrich trocknet deshalb von oben nach unten. Während die obere Schicht des Estrichs bereits nach mehreren Tagen nahe der Ausgleichsfeuchte liegt, dauert es in der mittleren Schicht mehrere Monate und im unteren Drittel sogar über ein Jahr. Mit der gängigen CM-Methode wird aber nur der durchschnittliche Feuchtegehalt des Estrichs ermittelt; es erfolgt keine tiefenaufgelöste Bestimmung des Feuchtegehalts.

Für den Belag des Estrichs mit einer weiteren Deckschicht ist es ausreichend, wenn der Estrich bis in eine bestimmte Tiefe getrocknet ist, sodass die getrocknete Estrichschicht einen höheren s_{d}-Wert (Wasserdampfdiffusionswiderstand) als der abdeckende Belag aufweist. Auf diese Weise kann nach oben immer mehr Feuchtigkeit entweichen, als von unten aus dem Estrich nachkommt.

Aus der DE 10 2013 018 917 A1 ist ein Verfahren zur Überwachung von Ingenieurbauwerken auf Feuchteschäden bekannt, bei welchem eine elektrolytisch wirksame Schicht in den Beton des Bauwerks eingearbeitet wird, deren Widerstand im Betrieb des Bauwerks fortlaufend überwacht werden kann.

Die nachveröffentlichte WO 2020/208471 A1 zeigt eine Vorrichtung zur Messung der Feuchte in Beton, insbesondere in bereits existierenden Bauwerken. Hierdurch soll eindringende Feuchte frühzeitig erkannt werden, ehe die Armierung korrodiert.

Die chinesische Gebrauchsmusterschrift CN 208060432 U offenbart eine Vorrichtung zur Bestimmung der Feuchte in einer Getreideschüttung.

Aus der Offenlegungsschrift CN 109752416 A ist ein Verfahren und eine Vorrichtung bekannt, mit welchen die Homogenität einer Emulsion bestimmt werden kann, beispielsweise eines Lackes.

Ausgehend vom Stand der Technik liegt der Erfindung somit die Aufgabe zugrunde, ein Verfahren zur zerstörungsfreien Messung der Feuchte in Beton und Estrich anzugeben, wobei der Trocknungsverlauf tiefenaufgelöst gemessen werden kann.

Die Aufgabe wird erfindungsgemäß durch ein Verfahren nach Anspruch 1 gelöst. Vorteilhafte Weiterbildungen der Erfindung finden sich in den Unteransprüchen. Erfindungsgemäß wird ein Verfahren zur Bestimmung des Wassergehaltes von Beton und Estrich vorgeschlagen, das sich durch die folgenden Verfahrensschritte auszeichnet:
- Einbringen von zumindest zwei ersten elektrischen Leitern, welche einen Abstand zueinander aufweisen, in eine erste Tiefe des Betons oder des Estrichs,
- Einbringen von zumindest zwei zweiten elektrischen Leitern, welche einen Abstand zueinander aufweisen, in eine zweite Tiefe des Betons oder des Estrichs, wobei die erste Tiefe von der zweiten Tiefe verschieden ist, wobei
- Die ersten und zweiten Leiter jeweils auf zumindest zwei Stäbe aufgebracht sind, und
- Messen des elektrischen Widerstandes zwischen den ersten und zwischen den zweiten elektrischen Leitern.

Das erfindungsgemäße Verfahren beruht also darauf, dass innerhalb des Betons oder Estrichs und somit in einer Schicht des Betons oder Estrichs, die durch die Eindringtiefe der beiden elektrischen Leiter in dem Beton oder Estrich definiert ist, der Wassergehalt und somit der Feuchtegrad innerhalb des Betons oder Estrichs bestimmt wird.

Die Widerstandsmessung kann in jeder beliebigen Art erfolgen, wie üblicherweise Widerstände gemessen werden. Beispiele dafür sind 4-Punkt-Messungen oder Messungen mit der Wheatstone'schen Messbrücke. Ferner können herkömmliche, auf Widerstandsmessung basierende Feuchtemessgeräte oder Ohmmeter eingesetzt werden, wie sie üblicherweise zur Messung des Feuchtegehalts einer Materialoberfläche bzw. eines elektrischen Widerstandes verwendet werden. Erfindungsgemäß wird der elektrische Widerstand zwischen Leitern gemessen, welche in der selben Tiefe des Betons oder des Estrichs angeordnet sind. Dabei ist die Tiefe der Abstand von der Oberfläche. Beton und insbesondere Estrich werden flächig eingesetzt, sodass sie eine gewisse Dicke aufweisen. Wie vorstehend beschrieben wurde, werden die ersten elektrischen Leiter in eine erste Tiefe und die zweiten elektrischen Leiter in eine zweite Tiefe eingebracht, wobei die erste Tiefe von der zweiten Tiefe verschieden ist. Durch die jeweilige Tiefe, d.h. den Abstand von der Oberfläche, wird eine Schicht innerhalb der Beton- oder Estrich-Fläche definiert, welche parallel zur Oberfläche verläuft. Durch Verwendung mehrerer Leiter in unterschiedlicher Tiefe ist es möglich, in mehreren Schichten des Betons oder des Estrichs den Wassergehalt zu messen.

Erfindungsgemäß werden zumindest zwei erste elektrische Leiter in einer ersten Tiefe des Betons oder des Estrichs und zumindest zwei zweite elektrische Leiter in eine zweite Tiefe des Betons oder des Estrichs eingesetzt. Dies führt zu einer ortsaufgelösten Messung in den einzelnen Schichten in unterschiedlichen Tiefen, so dass eine genauere Bestimmung der Feuchte ermöglicht wird.

In einigen Ausführungsformen kann das Verfahren zur Bestimmung des Wassergehalts in mehr als zwei dieser Schichten vorgenommen werden, beispielsweise in drei oder vier solcher Schichten. Dies kann dadurch erreicht werden, dass jeweils Paare von elektrischen Leitern in jeweils solchen Tiefen eingebracht werden, dass dadurch unterschiedliche Schichten des Betons definiert werden. Dies soll beispielhaft an einer Messung des Wassergehalts in drei unterschiedlichen Schichten eines Estrichs veranschaulicht werden. Der Estrich kann in eine Oberflächenschicht, eine darunter liegende Mittelschicht und eine Grundschicht unterteilt werden. Für die Messung in der Oberflächenschicht kann die Länge bzw. Eindringtiefe der zumindest zwei ersten Leiter so gewählt werden, dass deren Enden in der Oberflächenschicht zu liegen kommen. In gleicher Weise kann die Länge bzw. Eindringtiefe der zumindest zwei zweiten Leiter so gewählt werden, dass deren Enden in der Mittelschicht des Estrichs zu liegen kommen. Schließlich kann die Länge bzw. Eindringtiefe der zumindest zwei dritten Leiter so gewählt werden, dass deren Enden in der Grundschicht zu liegen kommen. Dies bedeutet also, dass für eine Messung in zum Beispiel drei Schichten des Estrichs zumindest drei Paare von elektrischen Leitern eingesetzt werden, wobei die Tiefe jedes dieser Paare so gewählt wird, dass mit einem Paar elektrischer Leiter der Wassergehalt in einer Schicht gemessen werden kann.

In analoger Weise kann die Messung in vier oder mehr unterschiedlichen Tiefen des Betons oder Estrichs erfolgen, wenn vier oder mehr Paare von elektrischen Leitern in jeweils unterschiedlicher Eindringtiefe des Betons oder Estrichs eingebracht werden.

In einer Ausführungsform enthält ein Leiter ein Metall oder eine Legierung, beispielsweise Kupfer oder Aluminium. Optional kann das Metall isoliert sein, wobei die Isolierung so gewählt wird, dass die Bestimmung des Wassergehalts nicht beeinflusst wird. Hierzu kann ein Ende des Leiters ohne Isolierung ausgeführt sein.

Erfindungsgemäß sind die Leiter auf Stäbe aufgebracht. Ein Stab im Sinne der vorliegenden Beschreibung ist ein schmaler, langer, zumeist massiver Gegenstand, der steif ist und zumeist überwiegend gerade, wobei die Länge groß gegenüber seinen übrigen Abmessungen ist. Das Querschnittsprofil des Stabes ist nicht eingeschränkt. Es kann beispielsweise H-förmig, T-förmig, Doppel-T-förmig, L-förmig oder polygonal sein, oder auch kreisförmig oder oval. Der Stab kann einen Kunststoff enthalten oder daraus bestehen, beispielsweise Polypropylen, Polyethylen und Polytetrafluorethylen. Diese Materialien sind besonders günstig, da sie elektrisch nicht leitend sowie gegenüber Feuchtigkeit resistent sind und somit nicht quellen, sodass Kurzschlüsse vermieden werden. Ferner können sie nach Durchführung der Messung, wenn der Estrich oder der Beton ausgehärtet ist, in einfacher Weise, beispielsweise durch Absägen, Abschneiden oder Abzwicken, entfernt werden. Der elektrische Leiter kann beispielsweise durch Kleben auf den Stab aufgebracht werdenb. Hierzu kann der Stab einen Draht oder eine Metallfolie enthalten oder daraus bestehen oder in Form einer Metallbeschichtung, insbesondere als Teilbeschichtung, ausgeführt sein.

In einigen Ausführungsformen weist der elektrische Leiter an dem Ende, das sich nicht im Beton oder Estrich befindet, einen Kontaktpunkt auf, mit dem der Kontakt zum Messgerät für die Messung des elektrischen Widerstandes hergestellt werden kann.

Im erfindungsgemäßen Verfahren werden die Leiter in Beton und Estrich eingebracht. Sie können dort über einen längeren Zeitraum verbleiben, sodass über einen solchen Zeitraum die Messung des Wassergehalts im Beton und Estrich erfolgen kann, um somit den optimalen Zeitpunkt festzulegen, damit beispielsweise der Estrich mit einem Bodenbelag belegt werden kann.

Mit dem erfindungsgemäßen Verfahren, insbesondere in den vorstehenden Ausführungsformen, kann der Wassergehalt/die Feuchte im Beton und Estrich gemessen werden, wobei der Trocknungsverlauf tiefenaufgelöst bestimmt werden kann. Ferner sind die für die Durchführung des erfindungsgemäßen Verfahrens benötigten Komponenten einfach handzuhaben und günstig herzustellen.

Das erfindungsgemäße Verfahren kann überall eingesetzt werden, wo Beton und Estriche genutzt werden. Das kann beispielsweise Ortbeton sein, um zu verhindern, dass dieser bei zu hohen Außentemperaturen zu schnell trocknet oder in zu feuchtem Zustand mit Bodenbelägen oder Überkonstruktionen belegt wird, beispielsweise einer Fußpfette.

Zur Durchführung des Verfahrens eignet sich eine Vorrichtung zur Bestimmung des Wassergehalts von Beton oder Estrich, wobei die Vorrichtung mindestens zwei Stäbe mit jeweils einem ersten Ende und einem gegenüberliegenden zweiten Ende umfasst, wobei auf jedem Stab zumindest ein erster elektrischer Leiter aufgebracht ist und zumindest auf einem Stab zumindest ein zweiter elektrischer Leiter aufgebracht ist, wobei der Abstand des Endes des ersten elektrischen Leiters vom ersten Ende der Stäbe verschieden ist vom Abstand des Endes des zweiten elektrischen Leiters vom ersten Ende des Stabes, und wobei die ersten und zweiten Leiter jeweils einen Kontaktpunkt zur Messung des elektrischen Widerstands aufweisen.

Zur Erläuterung der Bauweise dieser Vorrichtung sowie deren Funktionsweise wird auf vorstehende Ausführungen zum erfindungsgemäßen Verfahren verwiesen, denen entsprechende Informationen entnommen werden können.

In einer Ausführungsform sind mehrere solcher Stäbe mit jeweils einem, zwei oder mehreren elektrischen Leitern über Verbindungselemente miteinander verbunden. Auf diese Art und Weise kann eine Vorrichtung aus mehreren mit elektrischen Leitern versehenen Stäben bereitgestellt werden, mit denen in unterschiedlichen Tiefen der Wassergehalt gemessen werden kann. Da durch die Verbindungselemente der Abstand der Leiter innerhalb der jeweiligen Schichten konstant gehalten werden kann, ist eine einfache Kalibrierung möglich. Beispielsweise kann die Vorrichtung aus 2 bis etwa 10 oder 3 bis etwa 5 oder 4 Stäben bestehen, die über Verbindungselemente miteinander verbunden sind.

Die Verbindungselemente können ferner ebenfalls die Form eines Stabes aufweisen, wobei hierzu auf vorstehende Ausführungen in Zusammenhang mit dem Stab mit den beiden elektrischen Leitern verwiesen wird.

Die Vorrichtung kann vom Bodenleger beispielsweise während der Estrichverlegung in den feuchten Estrich eingebracht und dort belassen werden. Dabei befindet sich das erste Ende des Stabes mit den beiden elektrischen Leitern im Estrich, wohingegen die Verbindungselemente und die Kontaktpunkte zur Messung des elektrischen Widerstandes aus dem Estrich herausragen. Wenn der Estrich begehbar ist, kann über diese Kontaktpunkte die Estrichfeuchte in definierten Tiefen über den elektrischen Widerstand gemessen werden, wozu vorstehend genannte Messgeräte zur Messung des Widerstandes verwendet werden können.

Je nach Bedarf, Dicke des Estrichs und benötigter Informationen über den Massegehalt, kann die Vorrichtung aus zwei, drei, vier oder auch mehr solcher Stäbe bestehen, an denen jeweils in einer definierten Länge die elektrischen Leiter aufgebracht sind. Beispielsweise können die elektrischen Leiter an jeweils zwei unterschiedlichen Stäben günstigerweise gleich lang sein, sodass über Kontaktpunkte an der Oberseite der Komponenten die Widerstandsmessung für eine bestimmte Tiefe möglich ist. Da sich der Abstand der Kontaktpunkte an den verfügbaren Messgeräten orientiert (üblicherweise beträgt der Abstand der beiden Messdorne ca. 3 cm), sind die Abmessungen der Komponenten davon abhängig. Die Größe kann beispielsweise im Bereich von 100 mm x 40 mm x 40 mm liegen.

Nachdem der Estrich seine Zielfeuchte erreicht hat und belegt werden kann, kann der obere, aus dem Estrich herausragende Teil der Vorrichtung entfernt werden, beispielsweise durch Absägen, Abschneiden oder Abzwicken, was durch den Einsatz von Kunststoffen als Material für den Stab besonders einfach erreicht werden kann.

In einigen Ausführungsformen der Erfindung ist für eine Fläche des Estrichs von etwa 5 m² bis etwa 15 m² oder von etwa 7 m² bis etwa 13 m² von etwa 8 m² bis etwa 12 m² oder von etwa 10 m² eine erfindungsgemäße Vorrichtung vorzusehen. Diese eine Vorrichtung kann ausreichend zur Bestimmung des Wassergehalts in diesem Bereich sein.

Nachfolgend soll die Erfindung anhand von Figuren ohne Beschränkung des allgemeinen Erfindungsgedankens näher erläutert werden. Dabei zeigt
Figur 1 eine Vorrichtung vor dem Einbau.
Figur 2 zeigt eine Vorrichtung, wie sie in einem Estrich eingelassen ist.
Figur 3 zeigt den erfindungsgemäß bestimmten Verlauf der Trocknung in drei verschiedenen Estrich-Schichten über der Zeit.

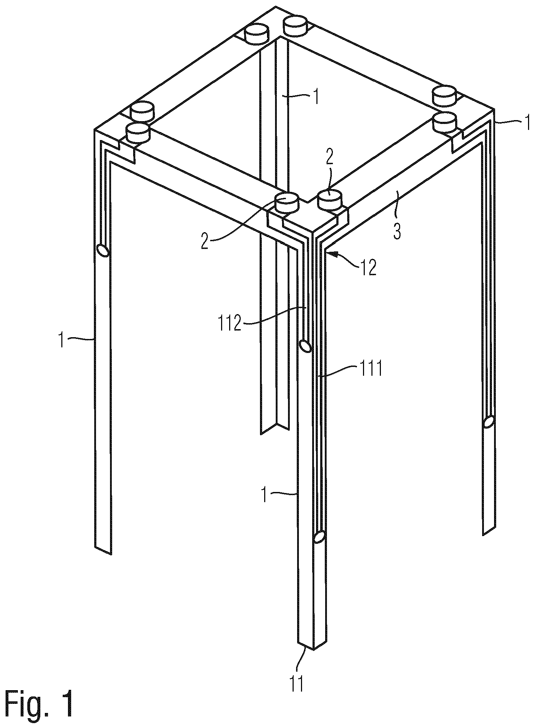

In Figur 1 ist eine Vorrichtung dargestellt, die insgesamt vier Stäbe 1 aufweist. Jeder dieser Stäbe 1 hat ein erstes Ende 11 und ein gegenüberliegendes zweites Ende 12. Die Stäbe sind aus einem Isolator gefertigt, beispielsweise einem Polymer, einem Thermoplast oder einem Duroplast.

Im dargestellten Ausführungsbeispiel weist die Vorrichtung vier Stäbe auf, wobei stets zwei Stäbe 1 am zweiten Ende 12 durch eines von vier Verbindungselementen 3 gleicher Länge miteinander verbunden sind. Bei der in Figur 1 dargestellten Ausführungsform sind die vier Stäbe 1 somit in Form eines Quadrates angeordnet. In anderen Ausführungsbeispielen kann die Vorrichtung auch zwei, drei oder mehr als vier solche Stäbe 1 aufweisen. Diese können am zweiten Ende 12 durch gerade oder gekrümmte Verbindungselemente 3 gleicher oder unterschiedlicher Länge miteinander verbunden sein.

In der dargestellten Ausführungsform ist jeder der sichtbaren Stäbe mit einem ersten elektrischen Leiter 111 und einem zweiten elektrischen Leiter 112 versehen. Die ersten und zweiten elektrischen Leiter sind jeweils aus einer Folie durch Schneiden oder Stanzen hergestellt und auf die Stäbe aufgeklebt. Die Folie kann ein Metall oder eine Legierung enthalten oder daraus bestehen. Aus Figur 1 ist deutlich erkennbar, dass der erste elektrische Leiter 111 an jedem Stab 1 kürzer ist als der zweite elektrische Leiter 112. In Figur 1 verdeckt ist ein Paar dritter elektrischer Leiter, welche nochmals länger sind als die zweiten elektrischen Leiter.

Wenn diese Vorrichtung mit den ersten Enden 11 der Stäbe 1 auf einer Rohdecke abgestellt und in einen Zementestrich eingegossen wird, wird aufgrund der unterschiedlichen Längen der beiden ersten und zweiten Leiter 111, 112 jeweils eine Schicht im Estrich definiert, welche einen definierten Abstand von der Oberfläche aufweist. Durch Widerstandsmessung an zwei benachbarten Kontaktpunkten 2 von zwei ersten Leitern 111 kann der Wassergehalt in dieser so definierten ersten Schicht gemessen werden. Durch Widerstandsmessung an zwei benachbarten Kontaktpunkten 2 von zwei zweiten Leitern 112 kann der Wassergehalt in der so definierten zweiten Schicht gemessen werden. Im dargestellten Ausführungsbeispiel sind die Kontaktpunkte 2 auf den Verbindungselementen 3 angeordnet, können sich aber auch am zweiten Ende 12 der Stäbe 1 befinden.

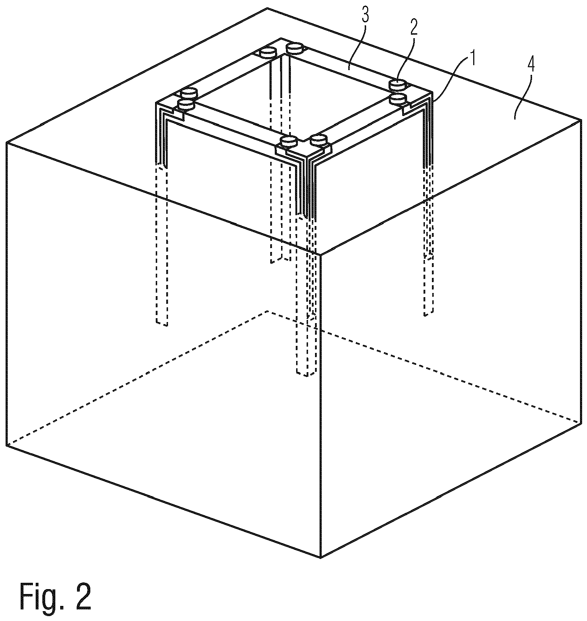

In Figur 2 ist die in der Figur 1 dargestellte Vorrichtung im Estrich 4 eingebracht. Identische Bauteile weisen dieselben Bezugszeichen auf, sodass auf die vorstehenden Ausführungen zu Figur 1 verwiesen wird.

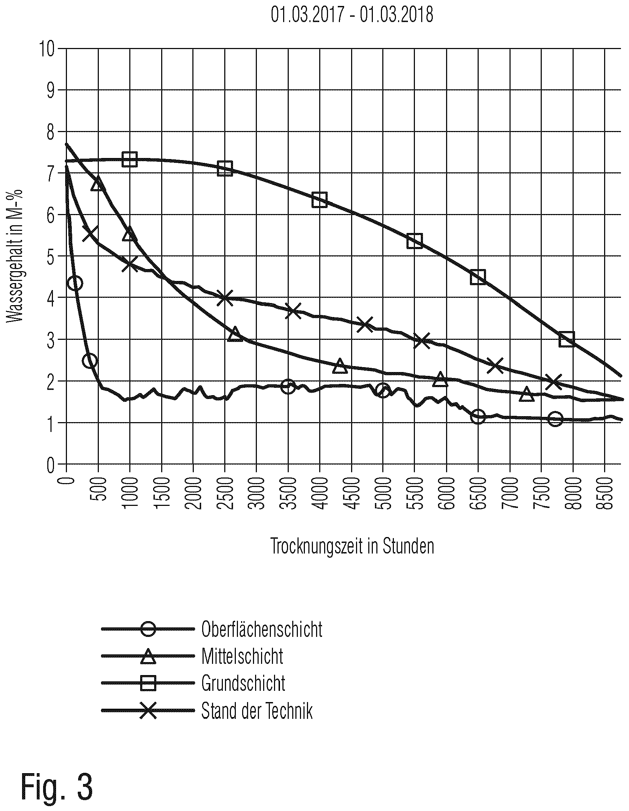

Figur 3 zeigt den Wassergehalt in verschiedenen Estrich-Schichten (Oberflächenschicht, Mittelschicht, Grundschicht) während einer Trocknungszeit von 8500 Stunden. Der Wassergehalt in diesen drei Schichten wurde mit einer Software simuliert und an diskreten Messpunkten mit einer Vorrichtung ermittelt, wie sie in den Figuren 1 und 2 beschrieben ist. Als Vergleich ist die herkömmliche Wassergehaltsbestimmung nach dem Stand der Technik angegeben. Bei diesem Stand der Technik handelt es sich um die Ermittlung der Estrich-Restfeuchte nach der sogenannten CM-Methode. Dabei wird ein Loch in den verlegten Estrich geschlagen oder gebohrt. Das so gewonnene Estrichmaterial wird dann in ein Calciumcarbid-Messgerät gegeben und über ein chemisches Verfahren die Rest-Feuchte ermittelt.

Es ist aus der Figur 3 erkennbar, dass mit dem erfindungsgemäßen Verfahren unter Verwendung der vorstehend erläuterten Vorrichtung die Rest-Feuchte im Estrich wesentlich genauer in Abhängigkeit der Tiefe bzw. der einzelnen Schichten gemessen werden kann. Im Gegensatz zum Verfahren aus dem Stand der Technik ist es mit dem erfindungsgemäßen Verfahren möglich, tiefenaufgelöst den Wassergehalt in der Estrichschicht zu bestimmen. Diese Messungen können erfindungsgemäß mit einfach handhabbaren und günstig hergestellten Messkomponenten durchgeführt werden.

Selbstverständlich ist die Erfindung nicht auf die in den Figuren dargestellten Ausführungsformen beschränkt. Die vorstehende Beschreibung ist daher nicht als beschränkend, sondern als erläuternd anzusehen. Die nachfolgenden Ansprüche sind so zu verstehen, dass ein genanntes Merkmal in zumindest einer Ausführungsform der Erfindung vorhanden ist. Dies schließt die Anwesenheit weiterer Merkmale nicht aus. Sofern die Beschreibung oder die Ansprüche "erste" und "zweite" Merkmale definieren, so dient dies der Unterscheidung gleichartiger Merkmale, ohne eine Rangfolge festzulegen.

## Patentansprüche

1. Verfahren zur Bestimmung des Wassergehalts von Beton und Estrich (4), mit folgenden Verfahrensschritten:
- Einbringen von zumindest zwei ersten elektrischen Leitern (111) in eine erste Tiefe des Betons oder des Estrichs (4), wobei die zwei ersten elektrischen Leiter (111) in der Ebene des Estrichs einen Abstand zueinander aufweisen, und wobei die erste Tiefe eine erste horizontale Schicht im Beton oder Estrich (4) definiert;
- Einbringen von zumindest zwei zweiten elektrischen Leitern (112) in eine zweite Tiefe des Betons oder des Estrichs (4), wobei die zwei zweiten elektrischen Leiter (112) in der Ebene des Estrichs einen Abstand zueinander aufweisen, und wobei die zweite Tiefe eine zweite horizontale Schicht im Beton oder Estrich (4) definiert, und wobei die erste Tiefe von der zweiten Tiefe verschieden ist;
- Messen des elektrischen Widerstandes zwischen den ersten elektrischen Leitern und zwischen den zweiten elektrischen Leitern,
- wobei die ersten und zweiten elektrischen Leiter (111, 112) jeweils auf zumindest zwei Stäbe (1) aufgebracht sind.

2. Verfahren nach Anspruch 1, wobei die Messung in drei oder vier Schichten des Betons und Estrichs (4) vorgenommen wird.

3. Verfahren nach einem der Ansprüche 1 bis 2, wobei die elektrischen Leiter (111, 112) ein Metall oder eine Legierung enthalten oder daraus bestehen.

4. Verfahren nach einem der Ansprüche 1 bis 2,
wobei die elektrischen Leiter (111, 112) Kupfer oder Aluminium enthalten oder daraus bestehen.

5. Verfahren nach einem der Ansprüche 1 bis 4, wobei die Stäbe (1) einen Kunststoff enthalten oder daraus bestehen.

6. Verfahren nach einem der Ansprüche 1 bis 5, wobei der erste elektrische Leiter (111) und der zweite elektrische Leiter (112) jeweils mit einem Kontaktpunkt (2) versehen sind, der sich außerhalb des Betons und des Estrichs (4) befindet und mit dem der Kontakt zum Messgerät für die Messung des elektrischen Widerstandes hergestellt wird.

7. Verfahren nach einem der Ansprüche 1 bis 6, wobei die Bestimmung des Wassergehalts über einen vorgebbaren Zeitraum erfolgt.

## Claims

1. Method for determining the water content of concrete and screed (4), comprising the following method steps:
- introducing at least two first electrical conductors (111) into a first depth of the concrete or the screed (4), the two first electrical conductors (111) being spaced apart from one another in the plane of the screed and the first depth defining a first horizontal layer in the concrete or screed (4);
- introducing at least two second electrical conductors (112) into a second depth of the concrete or the screed (4), the two second electrical conductors (112) being spaced apart from one another in the plane of the screed, and the second depth defining a second horizontal layer in the concrete or screed (4), and the first depth differing from the second depth;
- measuring the electrical resistance between the first electrical conductors and between the second electrical conductors,
- wherein the first and second electrical conductors (111, 112) are respectively applied to at least two bars (1).

2. Method according to claim 1, wherein the measurement is made in three or four layers of the concrete and screed (4).

3. Method according to any one of claims 1 to 2, wherein the electrical conductors (111, 112) contain or consist of a metal or an alloy.

4. Method according to any one of claims 1 to 2, wherein the electrical conductors (11 1, 112) contain or consist of copper or aluminum.

5. Method according to any one of claims 1 to 4, wherein the bars (1) contain or consist of a plastic material.

6. Method according to any one of claims 1 to 5, wherein the first electrical conductor (111) and the second electrical conductor (112) are each provided with a contact point (2) which is located outside the concrete and the screed (4) and with which contact is made with the measuring device for measuring the electrical resistance.

7. Method according to any one of claims 1 to 6, wherein the water content is determined over a predeterminable period of time.

## Revendications

1. Procédé pour déterminer la teneur en eau du béton et de la chape (4), comprenant les étapes suivantes consistant à :
- introduire au moins deux premiers conducteurs électriques (111) jusque dans une première profondeur du béton ou de la chape (4), les deux premiers conducteurs électriques (111) dans le plan de la chape présentant une distance l'un de l'autre, la première profondeur définissant une première couche horizontale dans le béton ou dans la chape (4) ;
- introduire au moins deux deuxièmes conducteurs électriques (112) jusque dans une deuxième profondeur du béton ou de la chape (4), les deux deuxièmes conducteurs électriques (112) dans le plan de la chape présentant une distance l'un de l'autre, la deuxième profondeur définissant une deuxième couche horizontale dans le béton ou dans la chape (4), la première profondeur étant différente de la deuxième profondeur ;
- mesurer la résistance électrique entre les premiers conducteurs électriques et entre les deuxièmes conducteurs électriques,
- les premiers et les deuxièmes conducteurs électriques (111, 112) étant appliqués respectivement sur au moins deux tiges (1).

2. Procédé selon la revendication 1,
dans lequel la mesure est effectuée dans trois ou quatre couches du béton et de la chape (4).

3. Procédé selon l'une des revendications 1 à 2,
dans lequel les conducteurs électriques (111, 112) contiennent ou consistent en un métal ou en un alliage.

4. Procédé selon l'une des revendications 1 à 2,
dans lequel les conducteurs électriques (111, 112) contiennent ou consistent en cuivre ou en aluminium.

5. Procédé selon l'une des revendications 1 à 4,
dans lequel les tiges (1) contiennent ou consistent en une matière plastique.

6. Procédé selon l'une des revendications 1 à 5,
dans lequel le premier conducteur électrique (111) et le deuxième conducteur électrique (112) sont chacun pourvus d'un point de contact (2) situé à l'extérieur du béton et de la chape (4) et permettant d'établir le contact avec l'appareil de mesure de la résistance électrique.

7. Procédé selon l'une des revendications 1 à 6,
dans lequel la détermination de la teneur en eau s'effectue sur une durée prédéfinissable.
